Europäisches Patentamt

European Patent Office

Office européen des brevets

(11) Publication number: 0 323 658 A2

# EUROPEAN PATENT APPLICATION

(21) Application number: 88202735.2

(51) Int. Cl.4: G01N 33/22 , G01N 31/12

(22) Date of filing: 30.11.88

(30) Priority: 05.12.87 NL 8702933
21.09.88 NL 8802336

(43) Date of publication of application:
12.07.89 Bulletin 89/28

(84) Designated Contracting States:
BE DE FR GB NL

(71) Applicant: N.V. NEDERLANDSE GASUNIE
Postbus 19
NL-9700 MA Groningen(NL)

(72) Inventor: Dijkstra, Kees
Troelstralaan 63D
NL-9722 JE Groningen(NL)
Inventor: Klimstra, Jacob
De Spil 8
NL-9285 VJ Buitenpost(NL)

(74) Representative: Bleukx, Lucas Lodewijk Maria,
Ir. et al
OCTROOIBUREAU DSM Postbus 9
NL-6160 MA Geleen(NL)

(54) Method for determining the wobbe number of a gas mixture.

(57) Method and device for determination of the Wobbe number of a gas mixture, in which the gas mixture is supplied to an oxidation gas, are supplied to a mixing chamber, a second gas mixture being formed in the mixing chamber, and this second gas mixture is burnt in a combustion chamber, after which the air factor of the combustion gas is measured. Depending on the air factor, the flow rate of the first gas mixture and/or the oxidation gas is set, and the ratio between the quantities used to set the flow rate of the first gas mixture and/or the oxidation mixture is determined.

FIG. 1

## METHOD FOR DETERMINING THE WOBBE NUMBER OF A GAS MIXTURE

The invention relates to a method for determining the Wobbe number of a gas mixture, which involves the supply of the gas mixture at a first flow rate from a supply section via at least one flow passage to a mixing chamber, and the supply of an oxidation gas at a second flow rate from a supply section via at least one flow passage to the mixing chamber, upon which a second gas mixture is formed in the mixing chamber, which second gas mixture is burnt in a combustion chamber and the air factor of the combustion oas is measured.

In mixing of flammable gases of varying origins, it is of great technical and economic importance to be able to determine the Wobbe number of the mixture in a fast, simple and inexpensive way. The Wobbe number is defined by the relationship

$$W = \frac{H}{\sqrt{d}}$$

where
H = gross calorific value of the gas in MJ per m³, and
d = density of the gas relative to air.

In fuel technology, the Wobbe number is an important quantity, because when heating gases that differ in composition but have the same Wobbe number are supplied at the same pressure to a gas-fired apparatus, the heat production remains the same. When, for instance in an industrial plant, a mixture of gases of varying origins is fired, for trouble-free firing the gases are to be mixed in such a ratio, if necessary with a different gas being added, as to produce a gas with a virtually constant Wobbe number.

From The Netherlands patent application 7808476 it is known that when gases of different compositions and Wobbe numbers are burnt with equal amounts of air, there is a direct correlation between the oxygen content of the flue gases and the relevant Wobbe number. It is not necessary to measure the Wobbe number as such: measurement of the oxygen content in said flue gases suffices.

The above method is known from European patent application 8151.

Starting from the measured air factor, it yields a direct indication of the Wobbe number of the first gas mixture.

In practice it is found that the sensors used for determining the air factor cannot as such be used for a direct indication of the Wobbe number. The measuring process generated by the customary sensor is temperature dependent and also varies with sensor age. Facilities to compensate for these variables can be incorporated in the measurement circuit, but this renders the overall measuring device complicated and less reliable.

The object of the invention is to provide a method of the above-mentioned type in which the drawbacks referred to are avoided.

According to the invention this object is achieved in that the flow rate of the first gas mixture and/or the oxidation gas is set in dependence of the air factor and in that the ratio between the quantities used for setting the flow rate of the first gas mixture and/or the oxidation gas is determined.

When this method is applied and the air factor is always controlled such that the second gas mixture meets the stoichiometric ratio, then the ratio as determined is a direct measure of the Wobbe number, which measure is independent of temperature influences and ageing of the sensor used. Temperature and ageing do affect the measurement curve of the sensor, but not the value measured in the stoichiometric point.

Other characteristics and advantages of the invention will become evident from the following description, in which reference is made to the attached drawings, where:

Figure 1 is a diagrammatical representation of a first device for carrying out the method according to the invention;

Figure 2 is a diagrammatical representation of a device for carrying out the method according to the invention in an embodiment that is changed relative to Figure 1;

Figure 3 is a diagrammatical representation of a device for carrying out the method according to the invention in an embodiment that is changed relative to the preceding figures;

Figure 4 is a diagrammatical representation of a device for carrying out the method according to the invention in an embodiment that is changed relative to the preceding figures;

Figure 5 is a diagrammatical representation of a device for carrying out the method according to the invention in an embodiment that is changed relative to the preceding figures.

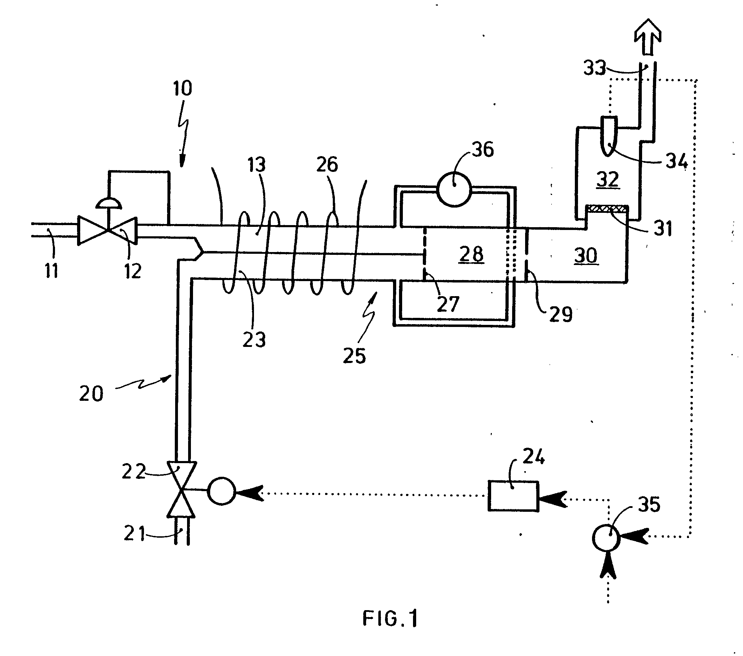

The device as shown in Figure 1 comprises a supply section 10 for the oxidation gas and a supply section 20 for the gas mixture of which the Wobbe number is to be determined. The oxidation gas may be air, but use may also be made of any other gas with which the gas mixture can be oxidized, such as pure oxygen, oxygen-enriched air, peroxides, etc.

The gas mixture may be natural gas, but in principle any other gas or gas mixture suitable for combustion purposes can be examined for its calorific values by means of the method according to the invention.

The oxidation gas supply section 10 comprises a supply line 11, which on one side is connected to an oxidation gas source supplying an oxidation gas at substantially constant pressure. On the other side supply line 11 is connected to a pressure control valve 12, via which the oxidation gas can flow into a pipe section 13. Via pressure control valve 12 the gas pressure in pipe section 13 is kept constant, for instance at a pressure of 4 bar.

The gas mixture supply section 20 comprises a supply line 21, which on one side is connected to a source of the gas mixture to be measured, and on the other side to a pressure control valve 22, through which the gas mixture can flow to a pipe section 23. The setting of pressure control valve 22 is determined by means of a control circuit 24, the operation of which will below be described in further detail.

Pipe sections 13 and 23 are at least in part parallel and in heat-exchanging contact. Around this parallel section 25, an electric heating coil has been fitted, which can be used to heat the gas mixture and the oxidation gas to the same temperature. Pipe sections 13 and 23 end in a joint baffle 27, in which for each pipe section at least one outlet is provided. Baffle 27 constitutes one end of a substantially cylindrical mixing chamber 28, the other end of which is bounded by a baffle 29. Baffle 29 has been provided with an outlet through which the second gas mixture, formed in mixing chamber 28 by mixing of the first gas mixture and the oxidation gas, can flow to a combustion chamber 30 with burner 31. Through combustion the flue gases escape into a chamber 32, which can be discharged via channel 33. In chamber 32 a λ probe 34 is installed, the measurement signal of which is fed back to control circuit 24. λ probe 34 determines the oxygen content in chamber 32. This λ probe 34 may be of the type described in a publication No. 730575 of Research Laboratories + General Motors Corp., Sensor for On-Vehicle Detection of Engine Exhaust Gas Composition, by Willium J. Fleming, David S. Howarth and David S. Eddy. Via a set point controller 35 the signal from the λ probe is fed back to control circuit 24, which in principle comprises a proportional integrating control for valve 22.

Between supply section 13 and supply section 23 a pressure gauge 36 is provided, which determines the pressure difference between the oxidation gas and the first gas mixture.

The device described operates as follows. Air with a pressure of for instance 6 bar is supplied through line 11. In pressure control valve 12 the air pressure is reduced to for instance 4 bar, following which the air, after heating, flows through the openings in baffle 27. A gas mixture with a constant pressure of for instance 6 bar is supplied through line 21. Via pressure control valve 22 the pressure of the gas mixture is reduced to about 4 bar, the correct value being dependent on the setting determined by control circuit 24. After heating, the gas mixture subsequently flows through the openings in baffle 27.

The openings in baffle 27 for the oxidation gas and the gas mixture, respectively, are chosen such that in principle their Reynolds number is the same and that the pressure drop for a gas mixture with an average to be expected Wobbe number equals the pressure drop for the oxidation gas. The opening in baffle 29 is chosen such that a sonic flow is created between mixing chamber 28 and combustion chamber 30, so that the flow rate is determined solely by the pressure, temperature and composition in the mixing chamber, and does no longer depend on the ambient pressure.

When, during operation, λ probe 34 finds an oxygen content of the combustion gas that is higher than the value set by set point controller 35, this means that the Wobbe number of the gas mixture is lower than that of the gas mixture used for system calibration. This implies that an additional amount of gas mixture is to be supplied in combustion chamber 30 in order to achieve the desired oxygen content. To this end, valve 22 is opened further via controller 24, as a result of which the amount of gas mixture in combustion chamber 30 increases and the excess air in chamber 32 decreases. By further opening of valve 22 the pressure in supply line 23 rises, so that a pressure difference is formed in gauge 36. In an equilibrium situation this is a direct indication of the Wobbe number of the gas mixture.

When the oxygen content in chamber 32 is too low, this means the Wobbe number of the gas mixture is higher than expected. This implies that valve 22 can be closed further so as to adjust the oxygen content in chamber 32 to the set value. This reduces the pressure in supply line 25, creating a pressure difference across gauge 36 which, however, is of the opposite sign compared with the situation in which the Wobbe number is too low. To calibrate the unit, via line 21 a calibration gas of known Wobbe number can be supplied. The supply of calibration gas can be controlled by means of set point controller 35 and valve 22 in such a way that the oxygen content in chamber 32 corresponds with the stoichiometric value measured by λ probe 34. The set point can be chosen such that the λ probe is used in its maximum sensitivity range. Because of the configuration chosen, the system is rendered insensitive to ageing of the λ probe with attending changes in the measurement curve, for said probe is not used by itself.

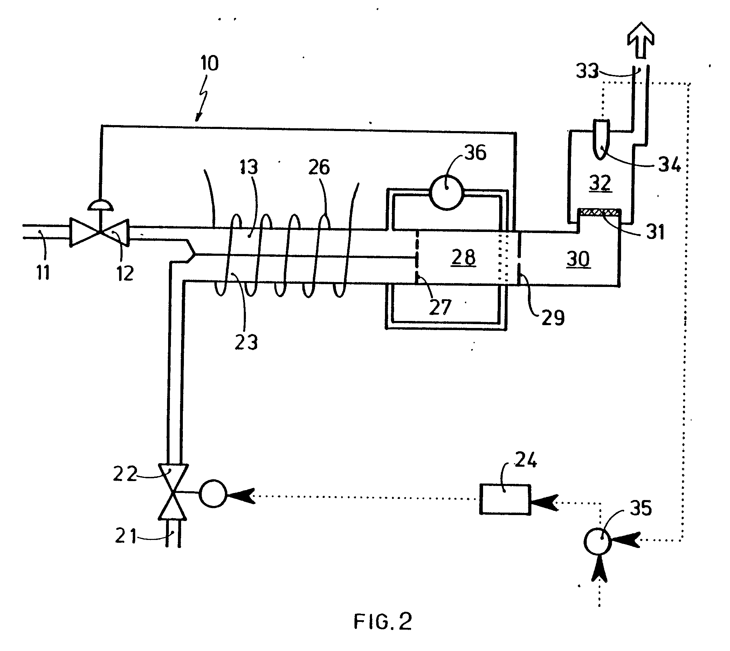

The embodiment shown in Figure 2 differs from that according to Figure 1 solely in the control of valve 12.

According to Figure 2 valve 12 is used to keep the pressure in mixing chamber 23 constant at for instance 4 bar. Thus, there is more certainty that a sonic flow is maintained between mixing chamber 28 and combustion chamber 30. Otherwise, operation of the device according to Figure 2 is identical with the operation of the device according to Figure 1.

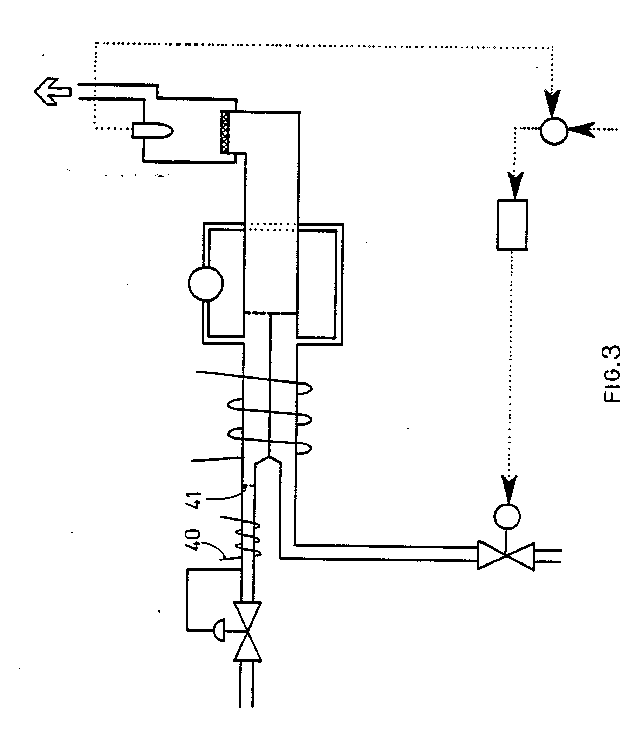

The embodiment shown in Figure 3 differs from that according to Figure 1 in that the supply line 13 for the oxidation gas is provided with a separate heating element 40 and a baffle 41 with a flow passage. The pressure before baffle 41 is controlled at 4 bar, while the oxidation gas is subjected to sonic expansion to a pressure of 1 bar through the opening in baffle 41. The heat exchanger 40 is needed to keep the temperature of the oxidation gas constant. The advantage of this embodiment is that the read-out of the Wobbe number is simplified, taking place at atmospheric pressure. Otherwise, operation is the same as that of the embodiment according to Figure 1.

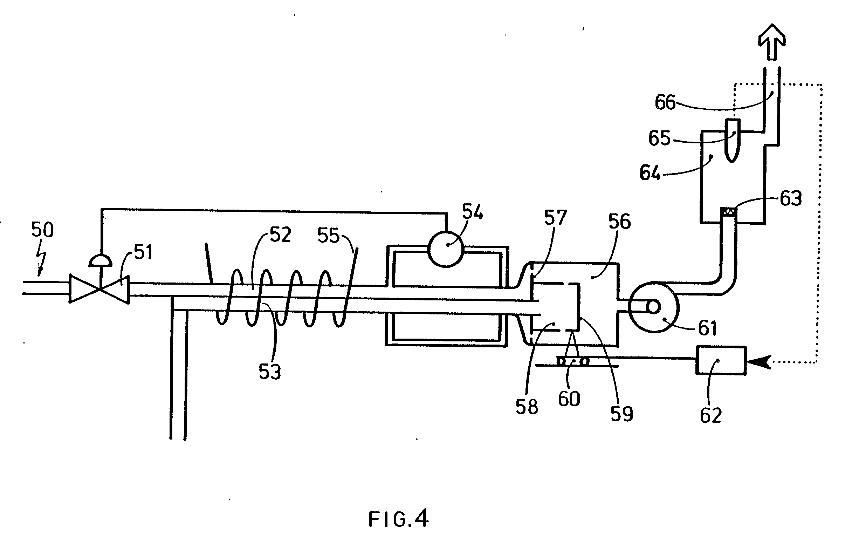

In the embodiment according to Figure 4 it is not the pressure of the gas mixture supplied that is controlled in dependence of the Wobbe number of said gas mixture, but it is the size of the outlet through which the oxidation gas flows to the mixing chamber that is controlled as a function of the Wobbe number.

The oxidation gas supply section is provided with a pressure control valve 51, which is used to keep the pressure in supply line 52 equal to the pressure of the oxidation gas supplied in line 53. This is effected by means of a pressure gauge 54, which measures the pressure difference between lines 52 and 53 and sets valve 51 such as to zero this pressure difference. The oxidation gas flows into a chamber 58, which includes a movable wall section 59, which can be used to adjust the size of the outlet openings between chamber 51 and mixing chamber 56. Via openings in wall 57 the gas mixture also flows into mixing chamber 56. By means of booster 61, gas mixture formed in mixing chamber 56 is sent to a burner 63, where it is burnt, the combustion gases being discharged via a channel 56. A λ probe 65 determines the oxygen content and can control the flow of oxidation gas into mixing chamber 56 via controller 62 and a trolley 60, which is coupled to wall section 59.

Operation of the device according to Figure 2 in principle is the same as that of Figures 1-3, it being understood that the setting of a valve is replaced by relocation of wall section 59, the actual position of the wall section being a measure of the Wobbe number of the gas mixture.

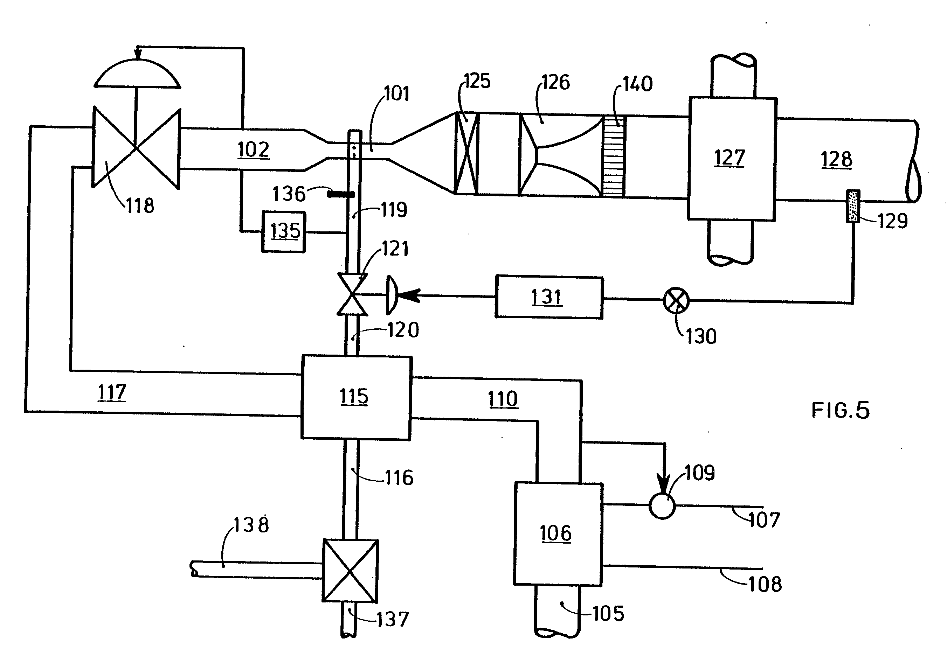

In the embodiment according to Figure 5, the central part of the Wobbe number measuring device is formed by a venturi system 101, the venturi operation of which is created by passing through of an oxidation gas, supplied via line 102. Venturi system is here understood to mean a lines system in which a local contraction has been made, which causes an underpressure that can be used to aspirate an other medium. Said contraction may be gradual, but stepwise contractions or other shapes are also possible. The oxidation gas may be air, but use may also be made of any other gas that is suitable for oxidation of the gas mixture to be measured, such as pure or enriched oxygen, peroxides, and the like. The oxidation gas is supplied at a pressure of for instance 4 bar via a line 105, passing first through a heat exchanger 106.

Here the oxidation gas is heated. The heat is preferably generated by means of electrical energy until a temperature between 30¤C and 40¤C is reached. The electrical energy is supplied via lines 107 and 108, line 107 being equipped with a controller 109, which is coupled to a temperature gauge in the outlet line 110 of heat exchanger 106. Via line 110 the oxidation gas passes to second heat exchanger 115, where it is brought into heat-exchanging contact with the gas mixture of which the Wobbe number is to be determined. This gas mixture is supplied to heat exchanger 115 via line 116. The oxidation gas leaves heat exchanger 115 via line 117 and passes into a control valve 118, from which it flows into line 102. By means of control valve 118 the pressure in line 102 is set at a constant value of for instance 3 bar. As already said, the gas mixture is supplied via line 116 through heat exchanger 115 and subsequently flows via line 120 through flow control valve 121, the operation of which will be explained later, and line 119 to the outlet openings in venturi system 101. Here, the gas mixture is "aspirated" due to the venturi effect in venturi system 101. During and after the outflow from venturi system 101, a second gas mixture is formed, consisting of the first gas mixture to which the oxidation gas is added. This gas initially flows through mixer 125, where the mixture consisting of the first gas mixture and the oxidation gas· is homogenized to a second gas mixture. Afterwards the second gas mixture flows through an expansion device 126, where supercritical expansion of the gas mixture takes place, following which the gas mixture enters a burner 40, where the second gas mixture is burnt. The combustion gas obtained is subsequently cooled in a cooler 127 so as to reduce its temperature to below 700¤C. Subsequently the combustion gas flows through line 128 to a further processing device or into the atmosphere. In line 128 a probe is present. This λ probe determines the oxygen content in line 128. Via a set point controller 130 the signal from the λ probe is fed back to a proportional integrating control 131, on the basis of which valve 121 is controlled. Between line 120 and line 102 a pressure gauge 135 is installed, which determines the pressure difference between the oxidation gas and the first gas mixture.

Operation of this system is as follows.
When the λ probe detects an oxygen content of the combustion gas that is higher than set in the set point controller, this means that the Wobbe number of the gas mixture is lower than that of the gas mixture used for calibration of the system.

This implies that extra gas is to be added in order to reach the desired oxygen content. To this end valve 121 is opened further, so that more gas mixture enters burner 140 and the oxygen excess in line 128 decreases. This results in a pressure rise in line 119, so that a pressure difference is formed across gauge 135. This thus is a direct indication of the Wobbe number of the gas mixture. When the oxygen content in line 128 is too low, this means that the Wobbe number of the gas mixture is higher than expected. This implies that valve 121 can be closed further so as to set the oxygen content in line 128 at the set value. This reduces the pressure in line 120, as a result of which a pressure difference is formed across gauge 125, which however is of the opposite sign compared with the situation when the Wobbe number is falling. To calibrate the unit, after line 138 a calibration gas with known Wobbe number may be employed. Line 119 is equipped with an adjustable constriction, which can be used to control the supply of calibration gas such that the oxygen content in line 128 corresponds with the value set in set point controller 130. In particular this constriction can be used in combination with the set point controller to obtain zero pressure difference across gauge 135 in a calibration gas situation. The set point can be chosen such that the λ probe is used in its maximum sensitivity range. The chosen configuration renders the system insensitive to ageing of the λ probe with attending changes in the measuring curve, for it is not used by itself.

The control point is not affected by ageing. Ageing does affect the slope of the curve, but this has influence only on the system's response time. However, this response time is always sufficient in the maximum sensitivity range and further does not affect the control point.

**Claims**

1. Method for determining the Wobbe number of a gas mixture, which involves the supply of the gas mixture at a first flow rate from a supply section via at least one flow passage to a mixing chamber, and the supply of an oxidation gas at a second flow rate from a supply section via at least one flow passage to the mixing chamber, upon which a second gas mixture is formed in the mixing chamber, which second gas mixture is burnt in a combustion chamber and the air factor of the combustion gas is measured, this method being characterized in that the flow rate of the first gas mixture and/or the oxidation gas is set in dependence of the air factor, and in that the ratio between the quantities used for setting the flow rate of the first gas mixture and/or the oxidation gas is determined.

2. Method according to claim 1, characterized in that the surface area of the passage through which the gas mixture flows from the supply section to the mixing chamber and/or the surface area of the passage through which the oxidation gas flows from the supply section to the mixing chamber is set as a function of the air factor measured, and in that the ratio between these two surface areas is determined.

3. Method according to claim 2, characterized in that the pressure of the gas mixture in the supply section is kept equal to the pressure of the oxidation gas in the supply section.

4. Method according to claim 1, characterized in that the pressure of the gas mixture in the supply section and/or the pressure of the oxidation gas in the supply section is set as a function of the air factor measured, and in that the pressure difference between the supply sections is determined.

5. Method according to claim 4 in which the pressure in only one of the two supply sections is controlled as a function of the air factor measured, characterized in that the pressure in the other supply section is kept constant.

6. Method according to claim 4, in which the pressure in only one of the two supply sections is controlled as a function of the air factor measured, characterized in that the pressure in the mixing chamber is kept constant by controlling the pressure in the other supply section.

7. Method according to any one of claims 1-5, characterized in that the oxidation gas is used as propellant for a venturi system, by which the first gas mixture is aspirated.

8. Method according to any one of the preceding claims, characterized in that the first gas mixture and the oxidation gas have been brought at the same temperature before they reach the mixing chamber.

9. Method according to any one of claims 1-8, characterized in that by preference the second gas mixture enters the combustion chamber after at least a supercritical expansion has taken place.

10. Device for carrying out the method according to any one of claims 1-9.

FIG.1

FIG.2

EP 0 323 658 A2

FIG.3

EP 0 323 658 A2

FIG.4

EP 0 323 658 A2

FIG.5

EP 0 323 658 A2